# EUROPEAN PATENT APPLICATION

(11) **EP 1 157 617 A2**
(43) Date of publication of application: **28.11.2001**
(21) Application number: 01111959.1
(22) Date of filing: 21.05.2001
(51) Int. Cl.: A23L 1/00

(54) **Use of alpha-keto enamine derivatives as ingredients**

(30) Priority: 23.05.2000 EP 00110886
(71) Applicant: Société des Produits Nestlé S.A., 1800 Vevey (CH)
(72) Inventor: Hofmann, Thomas, 85375 Neufahrn (DE); Ottinger, Harald, 80805 Munich (DE); Frank, Oliver, 85375 Neufahrn (DE); Soldo, Tomislav, 87700 Memmingen (DE); Cerny, Christoph, 1093 La Conversion (CH); Robert, Fabien, 01220 Divonne les Bains (FR); Blank, Imre, 1073 Savigny (CH)
(74) Representative: Thomas, Alain

(57) **Abstract**

The present invention concerns the use of a compound of general formula 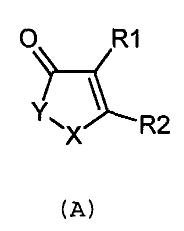 alone or in combination, as an ingredient for food, cosmetic, pharmaceutical and perfume compositions,
wherein
R1 is N-Pyrrolidinyl,
R2 is Methyl,
X is Ethyliden and
Y is Oxy radical.

## Description

The present invention concerns the use of alpha-keto enamine derivatives as ingredients.

The Maillard reaction of L-proline with reducing monosaccharides has been extensively studied during the last two decades in order to gain insights into the formation of volatiles during thermal processing of cereal products (Tressl et al., *J. Agric*. *Food Chem*. **1985a,** *33,* 919-923; Tressl et al. *J. Agric*. *Food Chem*. **1985b,** *33,* 924-928; Tressl et al. *J. Agric*. *Food Chem*. **1985c,** *33,* 1132-1137; Helak et al. *J. Agric*. *Food Chem*. **1989a,** *37,* 400-404; Helak et al. J. *Agric*. *Food Chem.* **1989b,** *37,* 405-410; Huyghues-Despointes et al. *J*. *Agric*. *Food Chem*. **1994,** *42,* 2519-2524).

By application of the GC/olfactometry techniques such as Charm analysis (Roberts, D.D., Acree, T. In *Thermally Generated Flavors;* Parliment T.H., Morello M.J., McGorrin R., Eds.; ACS, Washington DC, 1994, 71-79) or aroma extract dilution analysis (AEDA) (Hofmann, T., Schieberle, P. *J. Agric*. *Food Chem*. **1998,** *46,* 2721-2726), the odour-active compounds could be successfully detected in solvent extracts of Maillard reaction systems composed of L-proline and reducing sugars. Amongst the volatiles detected, the popcornlike smelling compounds 2-acetyl-1-pyrroline and 2-acetyltetrahydropyridine could be identified as the key contributors to the overall odour of thermally processed glucose/proline mixtures (Hofmann and Schieberle, *J. Agric. Food Chem*. **1998,** *46,* 2270-2277). Although the major part of the volatile reaction products formed during these roasting processes could be unequivocally shown by AEDA to have no odour activity, it can be not excluded that some of these odourless compounds might evoke a certain taste sensation on the tongue such as, e.g. bitterness, heating or cooling.

Consequently the sensory attributes of such reaction products from reducing carbohydrates and proline were characterised.

By application of the recently developed taste dilution analysis (Hofmann, T. *J. Agric*. *Food Chem*. **1999,** *47,* 4763-4768)
on HPLC fractions obtained from roasted glucose/proline mixtures two compounds could be detected, which showed an intense cooling effect on the tongue. These compounds were found to be formed in high concentrations when the hexose degradation product 2-hydroxy-3-methyl-2-cyclopenten-1-one was reacted in the presence of L-proline. After isolation by column chromatography both compounds could be obtained as pale-yellow oils with a purity of more than 99 %. GC/MS and 1D- and 2D-NMR spectroscopy led to the unequivocal identification of these cooling compounds as 5-methyl-2-(1-pyrrolidinyl)-2-cyclopenten-1-one (5-MPC) and 3-methyl-2-(1-pyrrolidinyl)-2-cyclopenten-1-one (3-MPC).

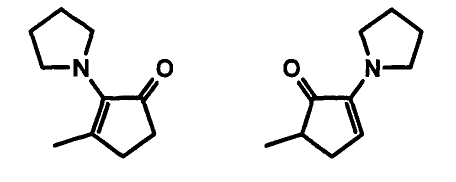

Although these compounds have been reported earlier by Tressl et al. (1985c) and Huyghues-Dispointes et al. (1994), these authors did not report on the cooling activity of these compounds when contacted with the tongue.

Besides 3-MPC and 5-MPC, we identified another cooling-active compound in the glucose/proline mixture, namely 2,5-dimethyl-4-(1-pyrrolidinyl)-3(2H)-furanone (DMPF).

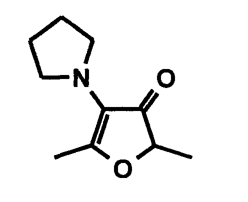

3-MPC as well as 5-MPC could easily be synthesised by heating 2-hydroxy-3-methyl-2-cyclopenten-1-one and pyrrolidinium acetate in ethanolic solution or by dry-heating of 2-hydroxy-3-methyl-2-cyclopenten-1-one in the presence of proline. In analogy, DMPF could be obtained by reacting 4-hydroxy-2,5-dimethyl-3(2H)-furanone in the presence of pyrrolidinium acetate in ethanol or in the presence of proline under dry-heating conditions, respectively.

The Japanese Patent 7242661 concerns DMPF as flavouring compound to impart flavour to wheat flour foods. This patent concerns flavour emitted from such foods which were just heated. Hence the patent deals with DMPF as flavour precursor. The GB Patent No. 1096427 concerns certain cyclopentanone derivatives as interesting compounds for perfumery. Certain alpha-keto enamines are claimed, but they are mentioned only as intermediates for the synthesis of other compounds.

The aim of the present invention is to have a group of compounds having excellent characteristics when used as an ingredient and more particularly as cooling agents in foods or other compositions. In contrast to menthol that exhibits a strong mint-like odour, these compounds possess no or only a faint odour and do not modify the aroma of a food or other compositions.

The object of the present invention is the use of a compound of the general formula 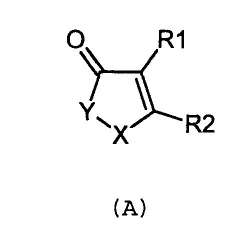 alone or in combination, as an ingredient for food, cosmetic, pharmaceutical and perfume compositions, wherein

R1 is taken from the group consisting of :
- N-Pyrrolidinyl
- N-Pyridinyl
- N-(amino-diethyl)
- N-(2-carboxy-pyrrolidinyl)
- N-(2-Methoxycarbonyl-pyrrolidinyl)

R2 is taken from the group consisting of :
- Hydrogen
- Methyl

X is taken from the group consisting of :
- Methylen
- Ethyliden
- 1-Propyliden
- Oxy radical

Y is taken from the group consisting of :
- Methylen
- Ethyliden
- 1-Propyliden
- Oxy radical
- Ethan-1,2-diyl
- Ethen-1,2-diyl
- Propan-1,2-diyl
- Ethan-1-oxy-1-yl.

In the present specification, alone means that only one compound of general formula (A) can be used. But, it is also possible according to the invention, to use several compounds of general formula (A) in the same composition.

In a first preferred embodiment of the general compound of the invention, R1 is N-Pyrrolidinyl, R2 is Hydrogen, X is Methylen and Y is Ethyliden (5-MPC). In a second preferred embodiment of the general compound of the invention, R1 is N-Pyrrolidinyl, R2 is Methyl, X is Oxy radical and Y is Methylen (MPF). In a third preferred embodiment of the general compound of the invention, R1 is N-Pyrrolidinyl, R2 is Methyl, X is Methylen and Y is Oxy radical (4-methyl-3-(1-pyrrolidinyl) -2 (5H) -furanone, 2(5H)MPF). In a fourth preferred embodiment of the general compound of the invention, R1 is N-Pyrrolidinyl, R2 is Methyl, X is Ethyliden and Y is Oxy radical (4,5-dimethyl-3-(1-pyrrolidinyl) -2 (5H) -furanone, 2(5H)DMPF).

In the case of a food composition, said composition is taken from the group consisting of chocolate, ice-cream, beverages, sugar confectionery, and petfood.

In the case of a cosmetic composition, said composition is taken from the group consisting of all topically usable cosmetic compositions.

In the case of a perfume composition, said composition is taken from the group consisting of alcohol-based or aqueous-based compositions.

The amount of the compound of general formula (A), alone or in combination is comprised between 0.01 and 3000 mg/kg from the whole composition. As already mentioned above, the above mentioned compounds of general formula (A) are used for the cooling effect they can induce to the compositions where they are introduced.

### General procedure for the synthesis of cooling substances of the general formula (A)

An ethanolic solution (e.g. 600 mL) of a cyclic enolone compound (e.g. 100 mmol) may be refluxed in the presence of equimolar amounts (e.g. 400 mmol) of an amino compound (e.g. pyrrolidine) and acetic acid for several hours (e.g. 1-5 h). After cooling to room temperature, the solvent may be removed in vacuo and the residue may be taken up in water. The pH may be adjusted to 10 with a sodium hydroxide solution (e.g. 30% in water). The solution may then be extracted with an organic solvent (e.g. diethyl ether), the combined organic layers washed with an aqueous solution of sodium carbonate (e.g. 200 mL; 0.5 mol/L), dried over sodium sulphate and then freed from solvent in vacuo. The target compounds may further be purified by column chromatography on aluminium oxide (basic, activity III-IV, Merck, Darmstadt, Germany). Chromatography may be performed using various organic solvents in different ratios such as for example hexane (e.g. 200 ml), hexane/diethyl ether (e.g. 7:3, 400 ml), hexane/diethyl ether (e.g. 3:7, 400 ml), and diethyl ether (e.g. 400 ml). The fraction obtained with diethyl ether may be freed from solvent *in vacuo* affording the target compound. The compounds in Table 1 can be synthesised according to this general procedure.

**Table 1.**

| **Cooling compounds synthesised** | | |
|---|---|---|
| **Enolones** | **Amines** | **Target compounds** |
| 2-Hydroxy-3-methyl-2-cyclopenten-1-one | Pyrrolidine | 3-Methyl-2-(1-pyrrolidinyl)-2-cyclopenten-1-one **(3-MPC)** 5-Methyl-2-(1-pyrrolidinyl)-2-cyclopenten-1-one **(5-MPC)** |
| 2-Hydroxy-3-methyl-2-cyclopenten-1-one | Piperidine | 3-Methyl-2-(1-piperidinyl)-2-cyclopenten-1-one **(3-MPipC)** 5-Methyl-2-(1-piperidinyl)-2-cyclopenten-1-one **(5-MPipC)** |
| 2-Hydroxy-3-methyl-2-cyclopenten-1-one | Diethylamine | 3-Methyl-2-diethylamino-2-cyclopenten-1-one **(3-MDeaC)** 5-Methyl-2-diethylamino-2-cyclopenten-1-one **(5-MDeaC)** |
| 2-Hydroxy-3-methyl-2-cyclopenten-1-one | L-Proline | 3-Methyl-2-(2-carboxy-1-pyrrolidinyl)-2-cyclopentene-1-one **(3-MProC)** |
| 2-Hydroxy-3-methyl-2-cyclopenten-1-one | L-Proline methylester | 5-Methyl-2-(2-methoxycarbonyl-1-pyrrolidinyl)-2-cyclo-pentene-1-one **(5-MMeproC)** |
| 2-Hydroxy-3-ethyl-2-cyclopenten-1-one | Pyrrolidine | 5-Ethyl-2-(1-pyrrolidinyl)-2-cyclopenten-1-one **(5-EPC)** |
| 2-Hydroxy-3,5-dimethyl-2-cyclopenten-1-one | Pyrrolidine | 3,5-Dimethyl-2-(1-pyrrolidinyl)-2-cyclopenten-1-one **(3,5-DMPC)** |
| 2-Hydroxy-3,4-dimethyl-2-cyclopenten-1-one | Pyrrolidine | 3,4-Dimethyl-2-(1-pyrrolidinyl)-2-cyclopenten-1-one **(3,4-DMPC)** 4,5-Dimethyl-2-(1-pyrrolidinyl)-2-cyclopenten-1-one **(4,5-DMPC)** |
| 2-Hydroxy-3-methyl-2-cyclohexen-1-one | Pyrrolidine | 3-Methyl-2-(1-pyrrolidinyl)-2-cyclohexen-1-one **(3-MPCH)** 6-Methyl-2-(1-pyrrolidinyl)-2-cyclohexen-1-one **(6-MPCH)** |
| 2,5-Dimethyl-4-hydroxy-3(2H)-furanone | Pyrrolidine | 2,5-Dimethyl-4-(1-pyrrolidinyl)-3(*2H*)-furanone **(DMPF)** |
| 4-Hydroxy-5-methyl-3(2*H*)-furanone | Pyrrolidine | 5-Methyl-4-(1-pyrrolidinyl)-3(*2H*)-furanone **(MPF)** |
| 3-Hydroxy-4,5-dimethyl-2(5*H*)-furanone | Pyrrolidine | 4,5-Dimethyl-3-(1-pyrrolidinyl)-2(5*H*)-furanone **(2(5H)-DMPF)** |
| 3-Hydroxy-4-methyl-2(5*H*)-furanone | Pyrrolidine | 4-Methyl-3-(1-pyrrolidinyl)-2(*5H*)-furanone **(2(5H) -MPF)** |

### Syntheses of 5-methyl-2-(1-pyrrolidinyl)-2-cyclopenten-1-one (5-MPC) and 3-methyl-2-(1-pyrrolidinyl)-2-cyclopenten-1-one (3-MPC)

***1. From 2-hydroxy-3-methyl-2-cyclopenten-1-on (cyclotene) and pyrrolidinium acetate in ethanol.*** A solution of cyclotene (100 mmol), pyrrolidine (400 mmol) and acetic acid (400 mmol) in ethanol (600 mL) was refluxed for 2 h. After cooling to room temperature, the solvent was removed in vacuo, the residue was taken up in water (300 mL) and the pH was adjusted to 10 with sodium hydroxide solution (30% in water). The solution was then extracted with diethyl ether (5 × 150 mL), the combined organic layers were washed with an aqueous solution of sodium carbonate (200 mL; 0.5 mol/L), dried over Na₂SO₄ and then freed from solvent in vacuo. The residual oil was dissolved in pentane/ethyl ether (6/4, v/v; 10 mL) and then applied onto a column (30 × 500 mm) filled with a slurry of aluminium oxide (basic, activity III-IV, Merck, Darmstadt, Germany) in pentane. Chromatography was performed using pentane (300 mL; fraction A), pentane/diethyl ether (9/1, v/v; 300 mL; fraction B), pentane/diethyl ether (8/2, v/v; 300 mL; fraction C), pentane/diethyl ether (7/3, v/v; 300 mL; fraction D), pentane/diethyl ether (6/4, v/v; 300 mL; fraction E), followed by pentane/diethyl ether (5/5, v/v; 300 mL, fraction F). Fraction B containing 5-MPC (1.65 g, 10 % in yield) and fraction D containing 3-MPC (1.32 g, 8 % in yield) were collected and freed from solvent under vacuo affording the target compounds as pale-yellow oils.

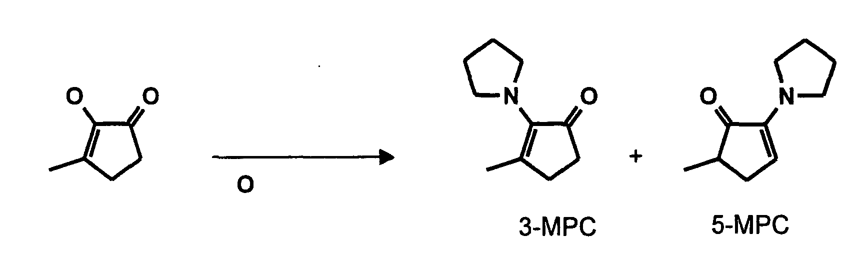

### Synthetic preparation of 3-methyl-2-(1-pyrrolidinyl)-2-cyclopentene-1-one (3-MPC) and 5-methyl-2-(1-pyrrolidinyl)-2-cyclopentene-1-one (5-MPC)

***2. From 2-hydroxy-3-methyl-2-cyclopenten-1-on (cyclotene) and proline dry-heated on aluminium oxide.*** A mixture of cyclotene (100 mmol) and proline (100 mmol) was ground with aluminium oxide (20 g, basic, activity III-IV) and then dry-heated for 10 min at 180°C. The mixture was suspended in water (100 mL) filtered, the pH was adjusted to 10 with sodium hydroxide solution (30 % in water) and then extracted with diethyl ether. The work-up of the reaction mixture was performed following the procedure detailed above for the cyclotene/pyrrolidinium acetate mixture. The target compounds 5-MPC (120 mg) and 3-MPC (33 mg) were obtained as pale-yellow oils.

### Spectroscopic data:

### 3-MPC:

MS (EI): 165 (100; [M]⁺), 164 (47), 137 (34), 136 (38), 122 (53), 109 (136), 108 (43), 94 (27), 81 (26), 67 (21), 41 (27).

¹H NMR (360 MHz; CDCl₃, COSY, TOCSY): δ 1.77-1.81 (m, 2×2H, CH₂), 2.13 (s, 3H, CH₃), 2.34-2.35 (m, 2H, CH₂), 2.39-2.41 (m, 2H, CH₂), 3.40-3.44 (m, 2×2H, CH₂).

¹³C NMR (360 MHz; CDCl₃; DEPT, HMQC, HMBC): δ 17.8 [CH₃], 24.9 [2×CH₂], 30.0 [CH₂], 34.1 [CH₂], 49.5 [2×CH₂], 143.7 [C], 145.9 [C], 205.9 [CO] .

### 5-MPC:

MS (EI): 165 (100; [M]⁺), 164 (32), 150 (26), 137 (22), 136 (37), 122 (87), 108 (34), 95 (34), 94 (31), 70 (21), 67 (24), 54 (24), 41 (25).

¹H NMR (360 MHz; CDCl₃, COSY, TOCSY) : δ 1.16-1.18 (d, 3H, J=7.5 Hz, CH₃), 1.82-1.88 (m, 2×2H, CH₂), 2.06-2.11 (dd, J=17.7, 2.2 Hz, 1H, CHₐH), 2.35-2.43 (m, 1H, J=7.5, 2.2; CH), 2.71-2.78 (dd, J=17.7, 3.1 Hz, 1H, CHH_{b}), 3.22-3.33 (m, 2×2H, CH₂), 5.82-5.83 (t, J=3.1 Hz, 1H, CH).

¹³C NMR (360 MHz; CDCl₃; DEPT, HMQC, HMBC): δ 16.5 [CH₃], 24.8 [2×CH₂], 32.6 [CH₂], 40.2 [CH], 48.1 [2×CH₂], 123.6 [CH], 146.7 [C], 207.4 [CO].

### Synthesis of 5-methyl-4-(1-pyrrolidinyl)-3(2H)-furanone (MPF)

***1*. *From xylose and pyrrolidine*.** A solution of xylose (0.1 mol) and pyrrolidine (0.1 mol) in methanol (90 ml) was refluxed for 3 h, then, acetic acid (0.1 mol) was added and heating was continued for additional 2 h. After cooling, the solvent was removed in vacuo, the residue was taken up in water (100 ml), extracted with ethyl acetate (100 ml, 5 times), and the combined organic layers were extracted with aqueous 0.1M sodium hydroxide solution (3 × 50 ml), the organic phase was dried (sodium sulphate) and fractionated by column chromatography using aluminium oxide (basic, activity III-IV; Merck, Darmstadt, Germany) conditioned in n-hexane. Chromatography was performed using hexane (200 ml), followed by hexane/diethyl ether 7:3 (400 ml); 3:7 (400 ml), and diethyl ether (400 ml). The fraction obtained with diethyl ether was freed from solvent *in vacuo* affording the target compound MPF (0.9 mmol; 0.9%) as colourless oil with a purity of more than 99%.

***2. From 4-hydroxy-5-methyl-3(2H)-furanone and pyrrolidine.*** A mixture of 4-hydroxy-5-methyl-2H-furan-3-one (10 mmol), pyrrolidine (20 mmol) and acetic acid (20 mmol) in methanol (50 ml) was refluxed for 3h. After cooling, the solvent was removed *in vacuo,* the residue was taken up in H₂O (100 ml), extracted with ethyl acetate (100 ml, 5 times), and the combined organic layers were extracted with aqueous 0.1M sodium hydroxide solution (3 x 50 ml), the organic phase was dried (sodium sulphate) and fractionated by column chromatography as detailed above. The target compound MPF (0.5 mmol; 5 % yield) was obtained as colourless oil with a purity of more than 99%.

### Spectroscopic data of MPF:

GC/MS (EI): 42 (100), 167 (95), 54 (93), 96 (76), 124 (74) ¹H NMR (360 MHz; CDCl₃, COSY, TOCSY) : 1.82 (m, 2×2H, CH₂), 2.23 (s, 3H, CH₃), 3.11 (m, 2×2H, CH₂), 4.38 (s, 2H, CH₂) ¹³C-NMR (360 MHz; CDCl₃) : 14.2 (CH₃), 24.7 (2×CH₂), 50.6 (2×CH₂), 72.9 (CH₂), 126.3 (C), 183.0 (C) , 198.9 (CO)

### Syntheses of 4.5-Dimethyl-3-(1-pyrrolidinyl)-2(5H)-furanone [2(5H)DMPF]

**From 3-Hydroxy-4,5-dimethyl-2(5H)-furanone and pyrrolidium acetate in ethanol.** A solution of 3-hydroxy-4,5-dimethyl-2(5H)-furanone (10 mmol), acetic acid (10 mmol) and pyrrolidine (10 mmol) in ethanol (50 mL) were refluxed for 3 h. After cooling to room temperature, the solvent was removed in vacuo and the residue was taken up in water (25 mL). The solution was then extracted with diethyl ether (5 x 10 ml), the combined organic layers were dried over sodium sulfate and then freed from solvent in vacuo. The residual oil was dissolved in pentane/diethyl ether (4/1, v/v; 5 mL) and then applied onto a column (30 x 500 mm) filled with a slurry of aluminium oxide (basic, activity III-IV, Merck, Darmstadt, Germany) in pentane. Chromatography was performed using pentane (300 mL; fraction A), pentane/diethyl ether (9/1, v/v; 400 mL; fraction B), pentane/diethyl ether (80/20, v/v; 400 mL; fraction C), pentane/diethyl ether (70/30, v/v; 400 mL; fraction D), pentane/diethyl ether (60/40, v/v; 400 mL; fraction E). Fraction E containing 2(5H)-DMPF (0.64 g, 36 % in yield) was collected and freed from solvent under vacuo affording the target compounds a colourless oil.

### Spectroscopic data of 2(5H)-DMPF:

MS (EI): 181 (82; [M]⁺), 166 (76), 138 (28), 136 (49), 122 (100), 110 (74), 108 (93), 94 (37), 82 (36), 68 (26), 55 (43), 54 (26), 53 (24), 43 (31), 41 (44).

¹H NMR (360 MHz; CDCl₃, COSY, TOCSY) : δ 1.35 - 1.36 (d, 3H, J=6.6 Hz, CH₃), 1.79 - 1.86 (m, 2x2H, CH₂), 2.03 (s, 3H, CH₃), 3.47 - 3.57 (m, 2x2H, CH₂), 4.66 - 4.72 (q, 1H, J=6.6 Hz, CH).

¹³C NMR (360 MHz; CDCl₃; DEPT, HMQC, HMBC) : δ 11.8 [CH₃], 19.2 [CH₃], 24.9 [2xCH₂], 49.3 [2xCH₂], 78.0 [CH], 128.7 [C], 130.5 [C], 170.4 [CO].

### Syntheses of 4-Methyl-3-(1-pyrrolidinyl)-2(5H)-furanone [2(5H)-MPF]

**From 4-Methyl-dihydro-furan-2,3-dione and pyrrolidium acetate in ethanol.** A solution of 4-methyl-dihydro-furan-2,3-dione (100 mmol, prepared according to Fleck et al., Helv. Chim. Acta **1950**, 33, 130), acetic acid (100 mmol) and pyrrolidine (100 mmol) in ethanol (225 mL) was refluxed for 2,5 h. After cooling down to room temperature, the solvent was removed in vacuo and the residue was taken up in water (200 mL). The solution was then extracted with diethyl ether (5 x 100 ml), the combined organic layers were dried over sodium sulphate and then freed from solvent in vacuo. The residual oil was dissolved in pentane/diethyl ether (3/2, v/v; 10 mL) and then applied onto a column (30 x 500 mm) filled with a slurry of aluminium oxide (basic activity III-IV, Merck, Darmstadt, Germany) in pentane. Chromatography was performed using pentane (300 mL; fraction A), pentane/diethyl ether (9/1, v/v; 400 mL; fraction B), pentane/diethyl ether (80/20, v/v; 400 mL; fraction C), pentane/diethyl ether (70/30, v/v; 400 mL; fraction D), pentane/diethyl ether (60/40, v/v; 400 mL; fraction E), pentane/diethyl ether (50/50, v/v; 400 mL; fraction F). Fraction F containing 2(5H)-MPF (2.25 g, 14 % in yield) was collected and freed from solvent under vacuo affording the target compound as a colourless oil.

### Spectroscopic data of 2(5H)-MPF:

MS (EI): 167 (94; [M]⁺), 166 (63), 139 (58), 138 (45), 122 (93), 120 (43), 111 (54), 110 (46), 108 (32), 95 (26), 94 (100), 82 (25), 81 (24), 80 (23), 68 (67), 67 (21), 55 (36), 54 (27), 53 (23), 41 (58) .¹H NMR (360 MHz; CDCl₃, COSY, TOCSY): δ 1.80 - 1.86 (m, 2x2H, CH₂), 2.09 (s, 3H, CH₃), 3.49 - 3.54 (m, 2x2H, CH₂), 4.53 (s, 2H, CH₂).¹³C NMR (360 MHz; CDCl₃; DEPT, HMQC, HMBC) : δ 12.4 [CH₃], 25.1 [2xCH₂], 49.5 [2xCH₂], 71.7 [CH₂], 124.3 [C], 130.7 [C], 171.6 [CO].

### Sensory analyses

Prior to sensory analysis, the purity of the synthetic taste compounds was checked by GC/MS. Determination of the cooling as well as the aroma threshold of the compounds was performed by trained panellists. Nasal odour thresholds (Guth, H.; Grosch, W. *J. Am. Oil Chem. Soc.* **1993,** *70,* 513-518) as well as cooling thresholds were determined by triangle tests using tap water as the solvent. The samples were presented in order of increasing concentrations and the threshold values evaluated in three separate sessions were averaged. The values between individuals and separate sessions differed by not more than one dilution step.

The results of the sensory analyses are summarised in Table 2. Besides menthol, 5-MPC, 2(5H)-DMPF, MPF and 2(5H)-MPF had the lowest cooling threshold. Comparison of the odour threshold concentrations revealed the lowest value for menthol eliciting a strong mint-like aroma, whereas the novel cooling agents showed significantly higher odour thresholds. Calculating the ratio of cooling threshold to odour threshold clearly demonstrated that the invention compounds, possessing no or only a faint odour, can be used as cooling compounds without imparting a strong odour. In comparison, for menthol the odour threshold is by a factor of 9.5 lower than the cooling threshold, thereby, indicating that it is hardly possible to evoke a cooling effect in a product without having a significant mint-like odour. These data also show that by using the novel cooling compounds, it is possible to evoke certain cooling effects during consumption of non-mint food compositions such as, confectionery products, malted beverages and fruity or brown flavours. Especially 2(5H)-DMPF, MPF and 2(5H)-MPF have a much lower ratio of cooling threshold to odour threshold as compared to menthol and are, therefore, very efficient cooling substances.

**Table 2.**

| **Comparison of cooling and odour thresholds of selected compounds** | | | | |
|---|---|---|---|---|
| Cooling substance | Cooling threshold^{a} | Odour threshold^{a} | Odour quality | Ratio (Cool/Odour ) |
| 3-MProC | 490 - 735 | --- | *odourless* | «0.01 |
| 5-MMeproC | 112 - 188 | --- | *odourless* | «0.01 |
| DMPF | 100 - 140 | 30 - 60 | nutty, roasty | 2.7 |
| 3-MPCH | 90 - 150 | 45 - 75 | faintly mint-like | 2.0 |
| 4,5-DMPC | 68 - 113 | 136 - 226 | faintly mint-like | 0.5 |
| 3-MPipC | 60 - 100 | 80 - 120 | faintly amine- | 0.8 |
| | | | like | |
| 3,4-DMPC | 51 - 86 | 26 - 43 | rubber-like | 2.0 |
| 3,5-DMPC | 33 - 54 | 16 - 27 | rubber-like | 2.0 |
| 3-MPC | 29 - 44 | 44 - 73 | faintly amine-like | 0.8 |
| 6-MPCH | 27 - 45 | 3.4 - 5.6 | rubber-like | 8.0 |
| 5-EPC | 27 - 43 | 13 - 22 | faintly mint-like | 2.0 |
| 5-MPipC | 16 - 24 | 12 - 20 | faintly mint-like | 2.7 |
| 5-MDeaC | 12 - 20 | 6.0 - 9.0 | curcuma-like | 2.1 |
| 5-MPC | 4.5 - 9 | 2.6 - 5.2 | faintly mint-like | 1.7 |
| 2(5H)-DMPF | 2.0 - 4.0 | 32 - 64 | faintly mint-like | 0.06 |
| MPF | 1.5 - 3.0 | --- | *odourless* | <<0.01 |
| 2(5H)-MPF | 0.02 - 0.06 | --- | *odourless* | <<0.01 |
| (-)-Menthol | 0.9 - 1.9 | 0.1 - 0.2 | mint-like | 9.5 |

| | | | | |
|---|---|---|---|---|
| ^{*a*} Threshold values [mg/kg] determined in water. | | | | |

In an additional experiment the cooling thresholds of 3-MPC, 5-MPC, MPF, 2(5H)-MPF and 2(5H)-DMPF have been determined in chocolate. As given in Table 3, also in chocolate the 2(5H)-MPF was evaluated with the lowest cooling threshold of about 0.25-0.5 mg/100g, whereas the 5-MPC showed an 8-fold higher cooling threshold.

**Table 3.**

| **Cooling thresholds of MPF, 3-MPC and 5-MPC in milk chocolate** | |
|---|---|
| Cooling compound | Cooling effect [mg/100g chocolate] |
| 2 (5H) -MPF | 0.25 -0.5 |
| MPF | 0.8 - 1.5 |
| 5-MPC | 2.3 - 3.7 |
| 2(5H)-DMPF | 5.0-7.5 |
| 3-MPC | 38 - 63 |

### Identification of 5-methyl-2-(1-pyrrolidinyl)-2-cyclopenten-1-one (5-MPC), 3-methyl-2-(1-pyrrolidinyl)-2-cyclopenten-1-one (3-MPC) and 2,5-dimethyl-4-(1-pyrrolidinyl)-3(2H)-furanone (DMPF) in roasted malt

Dark malt (50 g, Caraffa special) was frozen in liquid nitrogen and then ground in a mortar. The powder was then stirred overnight with dichloromethane (2 × 400 mL). The combined organic layers were then concentrated to about 50 mL in vacuo and the volatile fraction of the malt components were then isolated by high-vacuum distillation at 25°C. The distillate obtained was concentrated to about 1 mL and then fractionated by column chromatography (0.9 × 100 mm) on aluminium oxide (basic, activity III-IV, Merck, Darmstadt, Germany), which was conditioned in pentane. Chromatography was performed using pentane (100 mL; fraction A), pentane/diethyl ether (9/1, v/v; 100 mL; fraction B), pentane/diethyl ether (8/2, v/v; 100 mL; fraction C), pentane/diethyl ether (7/3, v/v; 100 mL; fraction D), pentane/diethyl ether (6/4, v/v; 100 mL; fraction E), pentane/diethyl ether (4/6, v/v; 100 mL; fraction F), pentane/diethyl ether (2/8, v/v; 100 mL; fraction G), followed by diethyl ether (100 mL, fraction H). Fraction B, fraction D and fraction G, respectively, were collected and analysed by GC/MS. By comparison of the retention times as well as mass spectra (EI, CI) with those obtained from the synthetic reference compounds, 5-MPC (101.3 µg/Kg) could be identified in fraction B, 3-MPC (9.4 µg/Kg) in fraction D and DMPF (11.5 µg/Kg) in fraction G.

### Example 1: Application of 5-methyl-2-(1-pyrrolidinyl)-2-cyclopenten-1-one (5-MPC) and 5-methyl-4-(1-pyrrolidinyl)-3(2H)-furanone (MPF) in mineral water

Solutions of 10% 1-menthol, 10% 5-MPC and 10% MPF, respectively, in ethanol were diluted with Vittel™ mineral water. The solutions were tasted and compared with pure Vittel™ water.

| Sample | Flavour |
|---|---|
| Vittel™ | Neutral in aroma and taste |
| 1-Menthol in Vittel™ (10 mg/L) | Pronounced cooling effect, strong mint-like odour |
| 5-MPC in Vittel™ (100 mg/L) | Pronounced cooling effect with a slight mint-like odour |
| MPF in Vittel™ (400 mg/L) | Pronounced cooling effect, neutral in aroma |

### Example 2: Application of 5-MPC and MPF in orange juice

Several cooling substances were evaluated in orange juice. 1-Menthol contributed with a strong cooling effect at 20 mg/L. It showed also a strong mint-like aroma, thus disbalancing the overall flavour of the fruit juice. The new cooling compound 5-MPC, added at 200 mg/L, exhibited also a strong cooling effect. In addition to its cooling effect, it exhibited a weak mint-like herbal aroma note, which was however less pronounced than with 1-menthol. The addition of 1000 mg/L MPF to orange juice caused a pronounced cooling effect in the mouth cavity without any additional aroma sensation. Hence, MPF is suitable to add a cooling effect to orange juice without changing the aroma profile, contrary to 1-menthol.

### Example 3: Application of 5-MPC and MPF in sugar confectionery

Sugar (100 g) was heated together with water (15 g) in a beaker on a hot plate. After the mixture became a clear solution, the sugar syrup was heated further until it became viscous due to the evaporation of water. Then 5-MPC (20 mg) and MPF (100 mg), respectively, were added. The viscous liquid was then poured into moulds (3 g each) and let cool down to room temperature. The candies, which had been formed in the moulds, were demoulded and used for taste testing. Both candies with 5-MPC and MPF, respectively, had a pronounced cooling effect in the mouth, compared with a reference without cooling substance. The candies with MPF showed no additional flavour quality and were preferred to those with 5-MPC, which were slightly oily and mint-like.

### Example 4: Application of 5-MPC in ice cream

Full-fat cream (250 g), milk (250 ml) and sugar (100 g) were mixed and stirred until the sugar was dissolved. Then the mixture was poured into the recipient of an ice cream machine (Il Gelataio Super, Simac Inc., Gessate, Italy) and frozen within 30 minutes while stirring. In the same way, ice cream was prepared with addition of 5-MPC (20 mg/kg). The ice cream with 5-MPC showed a pronounced long lasting cooling effect. The cool refreshing impression of this ice cream persisted much longer than with the unflavoured reference.

### Example 6: Topical testing of 5-MPC, 2 (5H)-DMPF, 2 (5H)-MPF and MPF

Topical thresholds of cooling compounds 5-MPF, 2(5H)-DMPF, 2(5H)-MPF and MPF were determined as follows: An aliquot (0.5 mL) of a solution, containing 0.05, 0.1, 0.2, 0.5, or 1.0% of the coolant in water, was applied to a circular area (10 cm²) of the skin surface on the inside of the right forearm, midway between the wrist and the elbow, and were rubbed for 1 min. In parallel, an aliquot (0.5 mL) of pure tap water was applied as the blank onto the skin of the left forearm. After 1 min, the skin was dried. A panel of 10 subjects (male and female) were asked to rank the cooling intensity on a scale from 0 (no effect) to 5 (very strong) . The values evaluated in three different sessions at two days were averaged. The values between individuals and separate sessions differed not more than 2 scores.

| **Topical testing of 5-MPC and MPF on the inside of the forearm** | | | | |
|---|---|---|---|---|
| Concentration [%] | Cooling intensity of^{*a*} | | | |
| | 2(5H)-MPF | 2(5H)-DMPF | MPF | 5-MPC |
| 0.00020 | 0 | | 0 | 0 |
| 0.00039 | 1 | | 0 | 0 |
| 0.00078 | 2 | | 0 | 0 |
| 0.00156 | 3 | | 0 | 0 |
| 0.00313 | 5 | | 0 | 0 |
| 0.00625 | 5 | 0 | 0 | 0 |
| 0.0125 | n.d. | 1 | 0 | 0 |
| 0.025 | n.d. | 3 | 0 | 0 |
| 0.05 | n.d. | 5 | 1 | 0 |
| 0.1 | n.d. | 5 | 2 | 0 |
| 0.2 | n.d. | 5 | 4 | 1 |
| 0.5 | n.d. | 5 | 5 | 3 |
| 1.0 | n.d. | 5 | 5 | 5 |

| | | | | |
|---|---|---|---|---|
| ^{*a*} Cooling effect on skin was determined in tap water by using a topical test scoring the cooling intensity on a scale from 0 (no effect) to 5 (very strong) . n.d. means cooling effect not detected. | | | | |

## Claims

1. Use of a compound of the general formula 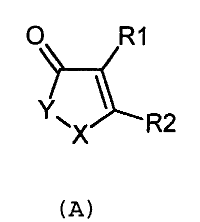 alone or in combination, as an ingredient for food, cosmetic, pharmaceutical and perfume compositions, wherein
R1 is taken from the group consisting of :
- N-Pyrrolidinyl
- N-Pyridinyl
- N-(amino-diethyl)
- N-(2-carboxy-pyrrolidinyl)
- N-(2-Methoxycarbonyl-pyrrolidinyl)
R2 is taken from the group consisting of :
- Hydrogen
- Methyl
X is taken from the group consisting of :
- Methylen
- Ethyliden
- 1-Propyliden
- Oxy radical
Y is taken from the group consisting of :
- Methylen
- Ethyliden
- 1-Propyliden
- Oxy radical
- Ethan-1,2-diyl
- Ethen-1,2-diyl
- Propan-1,2-diyl
- Ethan-1-oxy-1-yl.

2. Use according to claim 1, wherein R1 is N-Pyrrolidinyl, R2 is Hydrogen, X is Methylen and Y is Ethyliden.

3. Use according to claim 1, wherein R1 is N-Pyrrolidinyl, R2 is Methyl, X is Oxy radical and Y is Methylen.

4. Use according to claim 1, wherein R1 is N-Pyrrolidinyl, R2 is Methyl, X is Methylen and Y is Oxy radical.

5. Use according to claim 1, wherein R1 is N-Pyrrolidinyl, R2 is Methyl, X is Ethyliden and Y is Oxy radical.

6. Use according to any of claims 1 to 5, wherein the food compositions are taken from the group consisting of chocolate, ice-cream, beverages, sugar confectionery, and petfood.

7. Use according to any of claims 1 to 5, wherein the cosmetic compositions are taken from the group consisting of all. topically usable cosmetic compositions.

8. Use according to any of claims 1 to 5, wherein the perfume compositions are taken from the group consisting of alcohol-based or aqueous-based compositions.

9. Use according to any of claims 1 to 8, wherein the amount of the compound of general formula (A) alone or in combination is comprised between 0.01 and 3000 mg/kg from the whole composition.

10. Use of the compound of general formula (A), alone or in combination for the cooling effect.
